(19) Europäisches Patentamt
European Patent Office
Office européen des brevets

(11) Veröffentlichungsnummer : **0 232 820 B1**

# (12) EUROPÄISCHE PATENTSCHRIFT

(45) Veröffentlichungstag der Patentschrift : **08.01.92 Patentblatt 92/02**

(51) Int. Cl.[5] : **C07D 257/06**, C07D 257/04, C07C 323/60, C07C 323/12, C07C 331/04

(21) Anmeldenummer : **87101337.1**

(22) Anmeldetag : **31.01.87**

## (54) Thioether, Verfahren zu deren Herstellung sowie diese Verbindungen enthaltende Arzneimittel.

Die Akte enthält technische Angaben, die nach dem Eingang der Anmeldung eingereicht wurden und die nicht in dieser Patentschrift enthalten sind.

(30) Priorität : **08.02.86 DE 3604050**

(43) Veröffentlichungstag der Anmeldung : **19.08.87 Patentblatt 87/34**

(45) Bekanntmachung des Hinweises auf die Patenterteilung : **08.01.92 Patentblatt 92/02**

(84) Benannte Vertragsstaaten : **AT BE CH DE ES FR GB GR IT LI LU NL SE**

(56) Entgegenhaltungen :
**EP-A- 0 131 221**
**DE-A- 2 008 121**
**FR-A- 2 322 137**
**US-A- 3 536 661**

(56) Entgegenhaltungen :
**CHEMICAL ABSTRACTS, Band 100, Nr. 25, 18. Juni 1984, Seite 556, Ref.Nr. 209381c, Columbus, Ohio, US; & JP-A-58 222 064 (TAIHO PHARMACEUTICAL CO., LTD), 23-12-1983**
**Chem. soc. rev. (1979)18, "Medicinal Chemistry, Third Edition Part I A. Burger, Seiten 72-74"**

(73) Patentinhaber : **BOEHRINGER MANNHEIM GMBH**
**Sandhofer Strasse 116**
**W-6800 Mannheim 31 (DE)**

(72) Erfinder : **Reinholz, Erhard, Dr.**
**Kussmaulstrasse 9**
**W-6800 Mannheim 1 (DE)**
Erfinder : **Friebe, Walter-Gunar, Dr.**
**Sophienstrasse 8**
**W-6800 Mannheim 1 (DE)**
Erfinder : **Kampe, Wolfgang, Dr.**
**Wallstadter Strasse 3**
**W-6802 Ladenburg (DE)**
Erfinder : **Mertin, Jürgen, Dr.**
**Im Bangert 7**
**W-6149 Fürth-Ellenbach (DE)**
Erfinder : **Wilhelms, Otto-Henning, Dr.**
**Odenwaldstrasse 25/2**
**W-6941 Weinheim-Rittenweier (DE)**

**EP 0 232 820 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue Thioether, Verfahren zu deren Herstellung sowie diese Verbindungen enthaltende Arzneimittel.

Die erfindungsgemäßen Verbindungen können bei oraler und bei parenteraler Gabe anaphylaktische und anaphylaktoide Reaktionen hemmen, wie sie beispielsweise an sensibilisierten Meerschweinchen durch Allergenprovokation ausgelöst werden können. Außerdem wirken sie entzündungshemmend. Sie eignen sich daher zur Bekämpfung von allergischen Krankheiten, beispielsweise von allergischem Asthma.

Die vorliegende Erfindung betrifft neue Thioether der allgemeinen Formel I

$$Ar-O-A-\overset{(O)_n}{\overset{|}{S}}-B-R \qquad (I),$$

in welcher

Ar einen phenylrest, der gegebenenfalls ein-, zwei- oder dreifach durch $C_1$-$C_6$-Alkyl, $C_2$-$C_6$-Alkenyl, $C_1$-$C_6$-Alkoxy, $C_1$-$C_6$-Alkylthio, $C_1$-$C_6$-Alkanoyl, Hydroxyl, Hydroxy-$C_1$-$C_6$-alkyl, Carboxyl, $C_1$-$C_6$-Alkoxycarbonyl, Carbamoyl, Nitro, Amino und Halogen substituiert sein kann,

A einen geradkettigen oder verzweigten, gesättigten oder ungesättigten $C_1$-$C_8$-Alkylenrest, der gegebenenfalls durch eine Hydroxylgruppe substituiert sein kann,

n die Zahl 0, 1 oder 2,

B einen geradkettigen oder verzweigten, gesättigten oder ungesättigten $C_1$-$C_8$-Alkylenrest und

R 5-(1H)-Tetrazolyl, -CO-NH-Tetrazolyl, -CO-N(OH)-$R_1$, 5-(1H)-Tetrazolylthio, -C(=NH)-N(OH)$R_1$ oder -CO-O-NH-$R_1$ bedeuten, wobei

$R_1$ Wasserstoff, $C_1$-$C_6$-Alkyl, das gegebenenfalls durch $C_3$-$C_7$-Cycloalkyl substituiert sein kann, $C_2$-$C_6$-Alkenyl, $C_3$-$C_7$-Cycloalkyl, Phenyl-$C_1$-$C_6$-alkyl und phenyl darstellen, wobei der aromatische Teil durch $C_1$-$C_6$-AlkyL, $C_2$-$C_6$-Alkenyl, $C_1$-$C_6$-Alkoxy, $C_1$-$C_6$-Alkylthio, $C_1$-$C_6$-Alkanoyl, Hydroxyl, Hydroxy-$C_1$-$C_6$-alkyl, Carboxyl, $C_1$-$C_6$-Alkoxycarbonyl, Carbamoyl, Nitro, Amino und Halogen substituiert sein kann,

sowie deren pharmakologisch verträglichen Salze.

Analoge Verbindungen sind aus DE-A-33 24 916, EP-A-0 131 221 und US-A-3,536,661 bekannt. Insbesondere werden in EP-A-0 131 221 Verbindungen der Formel I beschrieben, in der R eine Hydroxygruppe darstellt. Im Gegensatz zu diesen zeigen die Verbindungen der vorliegenden Erfindung einen stärkeren Wirkungsgrad bei gleichem Dosisbereich.

Die zuvor genannten Alkylreste, allein oder in zusammengesetzter Form, enthalten jeweils 1 bis 6 Kohlenstoffatome und können geradkettig oder verzweigt sein.

Insbesondere bedeutet Alkyl die Mehyl-, Ethyl-, Propyl- und Butyl-Gruppe, Alkoxy die Methoxy- und Ethoxy-Gruppe, Alkylthio die Methylthiogruppe, Hydroxyalkyl die Hydroxymethyl-, Hydroxyethyl- Hydroxypropyl-, Hydroxybutyl-, Hydroxypentyl- und Hydroxyhexyl-Gruppe, wobei der Hydroxysubstituent an beliebiger Stelle der Alkylkette sich befinden kann, Alkoxycarbonyl die Methoxycarbonyl- und Ethoxycarbonyl-Gruppe.

Alkanoylgruppen sind bevorzugt Reste mit 1-4 C-Atomen, wie z.B. Formyl, Acetyl und Propionyl.

Cycloalkylreste sind bevorzugt Gruppen mit 3-7 C-Atomen, insbesondere Cyclopentyl und Cyclohexyl.

Die Aralkylgruppe ist insbesondere der Benzyl- und Phenethylrest.

Die Gruppe A stellt eine $C_1$-$C_3$-Alkylengruppe dar, bevorzugt mit 2-5 C-Atomen, wie z.B. Ethylen, Propylen und Butylen, wobei insbesondere die Propylengruppe einen Hydroxylsubstituenten trag kann. Als ungesaettigte Alkylengruppen kommen bevorzugt die Gruppen $-CH_2-CH=CH-CH_2-$ und $-CH_2-C\equiv C-CH_2-$ in Frage.

Die Gruppe B stellt eine $C_1$-$C_8$-Alkylengruppe dar, bevorzugt sind die Methylen-, Ethylen-, Propylen-, Butylen- und Pentylen-Gruppe. Als verzweigte Alkylenreste kommt bevorzugt die $-C(CH_3)_2-CH_2-$Gruppe in Frage. Ungesaettigte Alkylengruppen sind im besonderen die Gruppen $-CH_2-CH=CH-$ und $-CH_2-C\equiv C-$.

Der Alkenylrest enthaelt 2-6 Kohlenstoffatome, bevorzugt ist der Allylrest.

Als Halogenatome kommen Fluor, Chlor und Brom in Frage.

Bevorzugt kommen die Verbindungen in Frage, in denen Ar ein-, zwei- oder dreifach substituiert ist und Ar einen Methoxyphenyl-, Pentylphenyl-, Hydroxyhexylphenyl-, Dichlorphenyl-, 4-Acetyl-3-hydroxy-2-propylphenyl- und 4-Acetyl-2-allyl-3-hydroxyphenylrest darstellt, oder R eine N-Hydroxy-N-methyl-aminocarbonyl, N-Ethyl-N-hydroxy-aminocarbonyl-, N-Cyclohexyl-N-hydroxy-aminocarbonyl-, 5-Tetrazolylaminocarbonyl-, 5-Tetrazolyl-, 5-Tetrazolyl-thio- oder N-Hydroxy-aminocarbonylgruppe bedeutet.

Außer den in den Beispielen genannten Verbindungen sind Gegenstand der vorliegenden Erfindung insbesondere alle Substanzen, die jede mögliche Kombination der in den Beispielen genannten Substituenten aufweisen.

Bevorzugt sind folgende Verbindungen der Formel I, in der

Ar einen Phenylrest, der gegebenenfalls durch Propyl, Pentyl, Allyl, Methoxy, Ethoxy, Acetyl, Hydroxyl, 1-Hydroxy-hexyl und Halogen ein- bis dreifach substituiert sein kann,

A einen Methylen-, Ethylen-, Propylen-, Butylen-, Pentylen-, But-2-enylen-, But-2-inylen- und 2-Hydroxypropylenrest,

n die Zahl 0,

B einen Methylen-, Ethylen-, Propylen-, Butylen- oder Pentylenrest und

R eine 5-(1H)-Tetrazolyl-, -CO-NH-Tetrazolyl-, -CO-N(OH)-$R_1$, 5-(1H)-Tetrazolylthio- und -CO-O-NH-$R_1$ Gruppe bedeuten,

wobei $R_1$ Wasserstoff, eine Methyl-, Ethyl-, Propyl-, Cyclohexyl- und Phenylgruppe darstellt,

sowie deren pharmakologisch verträglichen Salze.

Besonders bevorzugt kommen Verbindungen der Formel I in Frage, in der

Ar einen Phenylrest, der durch Propyl, Allyl, Hydroxy, Acetyl und 1-Hydroxy-hexyl substituiert ist,

A einen Propylen-, Butylen-, But-2-enylen- und 2-Hydroxypropylen-Rest,

n die Zahl 0

B einen Methylen-, Ethylen-, Propylen- und Pentylenrest und

R eine N-Hydroxy-N-Methyl-aminocarbonyl-, 5-Tetrazolyl-aminocarbonyl-, 5-Tetrazolyl-, 5-Tetrazolylthio- oder N-Hydroxy-aminocarbonylgruppe

bedeuten, sowie deren pharmakologisch verträglichen Salze.

Weiterhin betrifft die Erfindung pharmazeutische Präparate mit einem Gehalt an Verbindungen der allgemeinen Formel I, sowie die Verwendung von Verbindungen der allgemeinen Formel I zur Herstellung solcher Präparate.

Das erfindungsgemäße Verfahren zur Herstellung von Verbindungen der Formel I ist dadurch gekennzeichnet, daß man in an sich bekannter Weise entweder

a) eine Verbindung der allgemeinen Formel II

$$\text{Ar-O-A-X} \qquad \text{(II)},$$

mit einer Verbindung der allgemeinen Formel III

$$\text{Y-B-G} \qquad \text{(III)},$$

worin

Ar, A und B die oben genannte Bedeutung haben, einer der beiden Reste X oder Y die Gruppe -SH, der andere einen mit der -SH-Gruppe reagierenden Rest darstellt und G für den oben genannten Rest R oder eine Hydroxylgruppe steht oder

b) eine Verbindung der allgemeinen Formel IV

$$\text{Ar-O-A-S-B-COOH} \qquad \text{(IV)},$$

in der

Ar, A und B die oben genannte Bedeutung haben, oder ein Carbonsäurehalogenid, Carbonsäureester oder gemischtes Carbonsäureanhydrid hiervon mit 5-Amino-(1H)-tetrazol oder mit einer Verbindung der allgemeinen Formel V

$$\text{HN(OH)-R1} \qquad \text{(V)},$$

oder einem O-silylierten Derivat davon, in welcher $R_1$ die oben genannte Bedeutung hat, umsetzt

und anschließend gewünschtenfalls

eine für G stehende Hydroxylgruppe, beispielsweise nach Überführung in ein Halogenid, Mesylat oder Tosylat in einen Rest R überführt,

eine Verbindung der Formel I, in der R für eine Cyano- oder Rhodanogruppe steht, mit Stickstoffwasserstoffsäure oder einem Alkaliazid und einer protonenliefernden Substanz umsetzt, und so Verbindungen erhält, in denen R die übrigen Bedeutungen besitzt,

das Schwefelatom oxidiert und das so erhaltene Reaktionsprodukt in ein pharmakologisch verträgliches Salz überführt.

Als reaktive Reste X und Y der Verbindungen II und III kommen Chlor, Brom, Mesyloxy oder Tosyloxy in Frage. Verbindungen der Formeln II und III, in denen Y die Gruppe -SH bedeutet, können als solche eingesetzt oder in situ aus geeigneten Vorstufen, wie beispielsweise einem S-alkyllierten 3-Thio-1,2-benzisothiazol-1,1-dioxid oder einem S-alkylierten Isothiuroniumsalz durch Basen freigesetzt werden.

Eine Umwandlung eines Restes R in einen anderen, durch den Anspruch definierten Rest R erfolgt beispielsweise durch Umsetzung einer Verbindung der Formel I, in der R für eine Cyanogruppe oder eine Rhodanogruppe steht, mit Stickstoffwasserstoffsäure oder ein Alkaliazid und einer protonenliefernden Substanz wie beispielsweise Ammoniumchlorid oder mit einer Verbindung der Formel V.

Die Ausgangsverbindungen der allgemeinen Formeln III und V sind literaturbekannte Substanzen oder koennen in Analogie zu literaturbekannten Verfahren hergestellt werden.

Die Herstellung der Verbindungen der allgemeinen Formeln II und IV ist aus EP 131 221 bekannt.

Das erfindungsgemaeße Verfahren fuehrt man beispielsweise so durch, daß man zunaechst eine Verbindung der allgemeinen Formel II, in der X einen reaktiven Rest bedeutet, mit einem Derivat des 3-Thio-1,2-benzisothiazol-1,1-dioxid, das am Schwefelatom den Rest -B-G traegt, in Gegenwart der aequimolaren Menge eines sekundaeren Amins umsetzt und das erhaltene Reaktionsprodukt isoliert.

Die Umsetzung erfolgt zweckmaeßig in einem polaren, aprotischen Loesungsmittel wie beispielsweise Acetonitril in Gegenwart eines tertiaeren Amins.

Gewuenschtenfalls kann das erhaltene Reaktionsprodukt in einem polaren, aprotischen Loesungsmittel mit einem Alkaliazid oder Ammoniumazid zur Reaktion gebracht werden.

Eine andere Variante besteht darin, eine Verbindung der allgemeinen Formel II, in der X die Gruppe -SH bedeutet, mit einer Verbindung der allgemeinen Formel III, in der Y einen reaktiven Rest darstellt, zu alkylieren.

Fuer den Fall, daß G eine Hydroxylgruppe bedeutet, kann das durch Umsetzung mit einer Verbindung der Formel II erhaltene Produkt beispielsweise durch Reaktion mit Triphenylphosphin und einem Tetrahalogenmethan in einem Halogenkohlenwasserstoff in ein Halogenid ueberfuehrt werden, aus dem durch Umsetzung in einem polaren, aprotischen Loesungsmittel wie beispielsweise Aceton mit einem Alkalicyanid oder Alkalirhodanid eine Verbindung der Formel I erhalten wird.

Eine Oxidation des Schwefelatoms in Verbindungen der allgemeinen Formel I kann beispielsweise dadurch erfolgent, daß man nach allgemein bekannten Methoden beispielsweise mit Sauerstoff, Wasserstoffperoxid, t-Butylhydroperoxid oder einer organischen Persaeure in einem Loesungsmittel wie Dichlormethan, Aceton oder Essigsaeure zum Sulfoxid oder Sulfon oxidiert.

Als pharmakologisch vertraegliche Salze kommen insbesondere Alkali-, Erdalkali- und Ammoniumsalze sowie gegebenenfalls Salze mit nicht toxischen anorganischen oder organischen Saeuren, wie z.B. Salzsaeure, Schwefelsaeure, Phosphorsaeure, Bromwasserstoffsaeure, Essigsaeure, Milchsaeure, Zitronensaeure, Äpfelsaeure, Benzoesaeure, Salicylsaeure, Malonsaeure, Maleinsaeure, Bernsteinsaeure oder Diaminocapronsaeure in Frage.

Die Salze erhaelt man in ueblicher Weise z.B. durch Neutralisation der Verbindungen der Formel I mit den entsprechenden Laugen oder Saeuren.

Zur Herstellung von Arzneimitteln werden die Verbindungen der allgemeinen Formel I in an sich bekannter Weise mit geeigneten pharmazeutischen Traegersubstanzen, Aroma-, Geschmacks- und Farbstoffen gemischt und beispielsweise als Tabletten oder Dragees ausgeformt oder unter Zugabe entsprechender Hilfsstoffe in Wasser oder Oel, wie z.B. Olivenoel, suspendiert oder geloest.

Die Substanzen der allgemeinen Formel I koennen in fluessiger oder fester Form oral und parenteral appliziert werden. Als Injektionsmedium kommt vorzugsweise Wasser zur Anwendung, welches die bei Injektionsloesungen ueblichen Stabilisierungsmittel, Loesungsvermittler und/oder Puffer enthaelt. Derartige Zusaetze sind z.B. Tartrat- oder Borat-Puffer, Ethanol, Dimethylsulfoxid, Komplexbildner (wie Ethylendiamintetraessigsaeure), hochmolekulare Polymer (wie fluessiges Polyethylenoxid) zur Viskositaetsregulierung oder Polyethylen-Derivate von Sorbitanhydriden.

Feste Traegerstoffe sind z.B. Staerke, Lactose, Mannit, Methylcellulose, Talkum, hochdisperse Kieselsaeure, hoehermolekulare Polymere (wie Polyethylenglykole).

Fuer die orale Applikation geeignete Zubereitungen koennen gewuenschtenfalls Geschmacks- und Sueßstoffe enthalten. Fuer die aeußerliche Anwendung koennen die erfindungsgemaeßen Substanzen I auch in Form von Pudern und Salben verwendet werden. Sie werden dazu z.B. mit pulverfoermigen, physiologisch vertraeglichen Verduennungsmitteln bzw. ueblichen Salbengrundlagen vermischt.

Die verabreichte Dosis haengt vom Alter, dar Gesundheit und dem Gewicht des Empfaengers, dem Ausmaß der Krankheit, der Art gleichzeitiger gegebenenfalls durchgefuehrter weiterer Behandlungen, der Haeufigkeit der Behandlungen und der Art der gewuenschten Wirkung ab. Ueblicherweise betraegt die taegliche Dosis der aktiven Verbindung 0.1 bis 50 mg/kg Koerpergewicht. Normalerweise sind 0.5 bis 40 und vorzugsweise 1.0 bis 20 mg/kg/Tag in einer oder mehreren Anwendungen pro Tag wirksam, um die gewuenschten Resultate zu erhalten.

Außer den in den nachstehenden Beispielen genannten Substanzen sind im Sinne der vorliegenden Anmeldung die folgenden Verbindungen bevorzugt:

N-Hydroxy-N-prop-2-enyl-S-[3-(4-acetyl-3-hydroxy-2-propylphenoxy)propyl]-3-mercaptopropionsaeure amid

5-[S-(4-<4-Acetyl-3-hydroxy-2-propyl-phenoxy>butyl)-2-mercaptoethyl]-(1H)-tetrazol

N-Hydroxy-S-[4-(4-acetyl-3-hydroxy-2-propyl-phenoxy)-butyl]-3-mercaptopropionsäureamidin

Beispiel 1

N-Hydroxy-N-methyl-S-[3-(4-acetyl-3-hydroxy-2-propyl-phenoxy)-propyl]mercaptoacetamid

2.6 g (8 mmol) S-[3-(4-Acetyl-3-hydroxy-2-propyl-phenoxy)propyl]-mercaptoessigsaeure werden in 30 ml Methylenchlorid geloest, mit 0.81 g (8 mmol) 4-Methylmorpholin versetzt und auf -10°C bis -15°C abgekuehlt. Bei dieser Temperatur wird innerhalb von 10 min eine Loesung von 1.15 g (8 mmol) Chlorameisensaeureisobutylester in 10 ml Methylenchlorid zugetropft. Anschließend wird noch 15 min bei gleicher Temperatur nachgeruehrt und dann mit 0.47 g N-Methylhydroxylamin in 10 ml Methylenchlorid versetzt.

Man laeßt noch 1 h bei -10°C und 2 h bei 0°C nachruehren und laeßt anschließend auf Raumtemperatur erwaermen. Dann wird das Methlyenchlorid abgezogen und der Rueckstand zweimal mit Ligroin ausgekocht. Der so erhaltene Rueckstand wird in Essigester geloest und dreimal mit 2 M NaOH extrahiert. Die waessrige Phase wird angesaeuert und dreimal mit Diethylether extrahiert. Zur Entfernung nicht umgesetzten Ausgangsmaterials wird der Ether dreimal mit 2 M $Na_2CO_3$-Loesung extrahiert.

Die Etherloesung wird anschließend mit Wasser, 2 M HCl und Wasser gewaschen, getrocknet und eingedampft. Man erhaelt 1.3 g (46 % Ausbeute) der Titelverbindung als Oel.

Beispiel 2

In analoger Weise wie in Beispiel 1 beschrieben erhaelt man

| | Bezeichnung | Ausbeute % | Schmp. (°C) Lsg.mittel |
|---|---|---|---|
| a) | N-Hydroxy-N-methyl-S-[3-(2-pentyl-phenoxy)propyl]-3-mercaptopropionsaeureamid | 60 | Oel |
| b) | N-Hydroxy-N-methyl-S-[3-(3-pentyl-phenoxy)propyl]-3-mercaptopropionsaeureamid | 50 | Oel |
| c) | N-Hydroxy-N-methyl-S-[4-(4-acetyl-3-hydroxy-2-propyl-phenoxy)butyl]-3-mercaptopropionsaeureamid | 71 | Oel |
| d) | N-Hydroxy-N-methyl-S-[3-(4-acetyl-3-hydroxy-2-propyl-phenoxy)propyl]-6-mercaptohexansaeureamid | 42 | Oel |
| e) | N-Hydroxy-N-methyl-S-[4-(4-acetyl-3-hydroxy-2-allyl-phenoxy)but-2-inyl)-3-mercaptopropionsaeureamid | 30 | Oel |
| f) | N-Hydroxy-N-methyl-S-[4-(4-acetyl-3-hydroxy-2-propyl-phenoxy)butyl]-mercaptoacetamid | 70 | Oel |
| g) | N-Hydroxy-N-methyl-S-[4-(acetyl-3-hydroxy-2-propyl-phenoxy)-but-2-inyl]-3-mercaptopropionsaeureamid | 32 | Oel |
| h) | N-Hydroxy-N-methyl-S-[3-(4-acetyl-3-hydroxy-2-allyl-phenoxy)propyl]-3-mercaptopropionsaeureamid | 35 | Oel |
| i) | N-Hydroxy-N-methyl-S-[3-(4-acetyl-3-hydroxy-2-propyl-phenoxy)propyl]-3-mercaptopropionsaeureamid | 40 | 112 - 9 (Ligroin Diethylether) |

Fortsetzung Beispiel 2

| | Bezeichnung | Ausbeute % | Schmp. (°C) Lsg.mittel |
|---|---|---|---|
| j) | N-Hydroxy-N-methyl-S-(3-phenoxy-propyl)-3-mercaptopropion-saeureamid | 85 | Oel |
| k) | N-Hydroxy-N-methyl-S-[3-(3-methoxy-phenoxy)propyl]-3-mercaptopropion-saeureamid | 65 | Oel |
| l) | N-Hydroxy-N-methyl-S-[3-(3-<1-hydroxy-hexyl>phenoxy)propyl]-4-mercaptobuttersaeureamid | 56 | Oel |
| m) | N-Hydroxy-N-methyl-S-[3-(3-<1-hydroxy-hexyl>phenoxy)-2-hydroxy-propyl]-4-mercaptobuttersaeure-amid | 65 | Oel |
| n) | N-Hydroxy-N-methyl-S-[4-(3-<1-hydroxy-hexyl>phenoxy)butyl]-3-mercaptopropionsaeureamid | 52 | Oel |
| o) | N-Hydroxy-N-methyl-S-[4-(3-<1-hydroxy-hexyl>phenoxy)-but-2-enyl]-3-mercaptopropionsaeure-amid | 15 | Oel |
| p) | N-Hydroxy-N-methyl-S-[3-(4-acetyl-3-hydroxy-2-propyl-phenoxy)-2-hydroxy-propyl]-3-mercaptopropionsaeureamid | 47 | Oel |
| q) | N-Hydroxy-N-ethyl-S-[3-(4-acetyl-3-hydroxy-2-propyl-phenoxy)-propyl]-3-mercaptopropionsaeure-amid | 68 | 56 (Ligroin) |

| | Bezeichnung | Ausbeute % | Schmp. (°C) Lsg.mittel |
|---|---|---|---|
| r) | N-Hydroxy-N-propyl-S-[3-(4-acetyl-3-hydroxy-2-propyl-phenoxy)-propyl]-3-mercaptopropionsaeure-amid | 77 | 57-60 (Ligroin) |
| s) | N-Hydroxy-N-phenyl-S-[3-(4-acetyl-3-hydroxy-2-propyl-phenoxy)propyl]-3-mercapto-propionsaeureamid | 70 | 64-66 (Ligroin) |
| t) | O-[S-(3-<4-acetyl-3-hydroxy-2-propyl-phenoxy>propyl)-3-mercapto-propionyl]cyclohexyl-hydroxylamin | 14 | Oel |
| u) | N-Hydroxy-N-methyl-S-[5-(4-acetyl-3-hydroxy-2-propyl-phenoxy)pentyl]-3-mercapto-propionsaeureamid | 31 | 76 (Ligroin) |
| v) | N-Hydroxy-N-methyl-S-[3-(3,4-dichlor-2-propyl-phenoxy)propyl]-3-mercaptopropionsaeureamid | 42 | 35-36 (Ligroin) |

Beispiel 3

N-[(1H)-Tetrazol-5-yl]-S-[3-(2-pentyl-phenoxy)propyl]-3-mercaptopropionsaeureamid

0.9 g (29 mmol) s-[3-(2-Pentyl-phenoxy)propyl]-3-mercaptopropionsaeure werden in 10 ml Methylenchlorid geloest und mit 0.29g(29 mmol) N-Methylmorpholin versetzt. Man kuehlt auf -10°C ab, und tropft bei dieser Temperatur 0.39 g (29 mmol) Chlorameisensaeureisobutylester in 5 ml Methylenchlorid zu. Man ruehrt noch 15 min und gibt danach eine Suspension von 0.3 g (29 mmol) 5-Amino-(1H)-tetrazol in 5 ml Methylenchlorid zu.

Man ruehrt 1 h bei -10°C, 1.5 h bei 0°C und laeßt dann auf Raumtemperatur erwaermen. Anschließend wird das Methylenchlorid abgedampft, der Rueckstand mit Essigester versetzt und mit 2 M HCl extrahiert. Der Essigester wird getrocknet und eingedampft.

Nach Chromatographieren an Kieselgel im System Ligroin/Essigester (1:2) mit 0.25 % Essigsaeure erhaelt man 0.4 g (37 % Ausbeute) der Titelverbindung von Schmelzpunkt 151-68°C.

Beispiel 4

In analoger Weise wie in Beispiel 1 beschrieben erhaelt man:

| Bezeichnung | Ausbeute % | Schmp. (°C) Lsg.mittel |
|---|---|---|
| N-[(1H)-Tetrazol-5-yl]-S-[3-(4-acetyl-3-hydroxy-2-allyl-phenoxy)propyl]-3-mercaptopropionsaeureamid | 25 | 209 (Methanol) |
| N-[(1H)-Tetrazol-5-yl]-S-[3-(4-acetyl-3-hydroxy-2-propyl-phenoxy)propyl]-6-mercaptohexansaeureamid | 40 | 190 - 3 (Essigester) |

| Bezeichnung | Ausbeute % | Schmp. (°C) Lsg.mittel |
|---|---|---|
| c) N-[(1H)-Tetrazol-5-yl]-S-[3-(3-<1-hydroxy-hexyl>phenoxy)propyl]-4-mercaptobuttersaeureamid | 40 | 175-82 (i-Propanol) |
| d) N-[(1H)-Tetrazol-5-yl]-S-[3-(3-<1-hydroxy-hexyl>phenoxy)-2-hydroxy-propyl]-4-mercaptobuttersaeureamid | 50 | 164-7 (Diethyl-ether) |
| e) N-[(1H)-Tetrazol-5-yl]-S-[4-(3-<1-hydroxy-hexyl>phenoxy)butyl]-3-mercaptopropionsaeureamid | 36 | 148-50 ($H_2O$) |
| f) N-[(1H)-Tetrazol-5-yl]-S-[4-(3-<1-hydroxy-hexyl>phenoxy)-but-2-enyl]-3-mercaptopropionsaeureamid | 15 | 145-8 ($CH_2Cl_2$, Methanol) |
| g) N-[(1H)-Tetrazol-5-yl]-S-[3-(3,4-dichlor-phenoxy)propyl]-3-mercapto-propionsaeureamid | 45 | 165-7 (Diethyl-ether) |
| h) N-[(1H)-Tetrazol-5-yl]-S-[4-(4-acetyl-3-hydroxy-2-propyl-phenoxy)-but-2-inyl]-3-mercaptopropionsaeure-amid | 43 | 178-81 (Diethyl-ether/ Aceton) |
| i) N-[(1H)-Tetrazol-5-yl]-S-[3-(4-chlor-phenoxy)propyl]-3-mercapto-propionsaeureamid | 55 | 160-62 (Wasser) |
| j) N-[(1H)-Tetrazol-5-yl]-S-[3-(4-chlor-3-trifluormethyl-phenoxy)-propyl]-3-mercaptopropionsaeure-amid | 68 | 174-76 (2-Propanol) |

Beispiel 5

5- <S-[3-(4-Acetyl-3-hydroxy-2-allyl-phenoxy)propyl]-mercaptomethyl>-(1H)-tetrazol

2.1 g (7 mmol) S-[3-(4-Acetyl)-3-hydroxy-2-allyl-phenoxy)-propyl]mercaptoacetonitril werden in 50 ml DMF geloest, mit 2.27 g (35 mmol) Natriumazid und 1.87 g (35 mmol) Ammoniumchlorid versetzt und 48 h bei 120°C und 150 bar Stickstoff geruehrt.

Anschließend wird das DMF abgedampft, der Rueckstand mit NaOH versetzt und abfiltriert. Die Loesung wird mit Methylenchlorid ausgeschuettelt, anschließend sauer gestellt und das Produkt mit Methylenchlorid extrahiert.

Nach Chromatographieren an Kieselgel im System Methylenchlorid/Methanol/Wasser (325:225:25) werden 0.6 g (25 % Ausbeute) der Titelverbindung vom Schmelzpunkt 129 - 32°C erhalten.

Beispiel 6

In analoger Weise wie in Beispiel 5 wurden erhalten

| Bezeichnung | Ausbeute % | Schmp. (°C) Lsg.mittel |
|---|---|---|
| 5-<S-[3-(4-Acetyl-3-hydroxy-2-propyl-phenoxy)propyl]-5-mercapto-pentyl>-(1H)-tetrazol | 28 | 93 ($CH_2Cl_2$, $CH_3OH$) |
| 5-<S-[3-(4-Acetyl-3-hydroxy-2-propyl-phenoxy)propyl]-2-mercaptoethyl>-(1H)-tetrazol | 41 | 126 ($CH_2Cl_2$, $CH_3OH$) |
| 5-<S-[3-(2-Pentyl-phenoxy)propyl]-2-mercaptoethyl>-(1H)-tetrazol | 36 | 51 (Ligroin) |
| 5-<S-[3-(4-Acetyl-3-hydroxy-2-allyl-phenoxy)propyl]-2-mercaptoethyl>-(1H)-tetrazol | 32 | 112 - 9 ($H_2O$) |

Beispiel 7

<S-[3-(4-Acetyl-3-hydroxy-2-propyl-phenoxy)propyl]-2-mercaptoethyl>-5-mercapto-(1H)-tetrazol

0.7 g (2 mmol) S-[3-(4-Acetyl-3-hydroxy-2-propyl-phenoxy)-propyl]-2-mercaptoethylrhodanid werden in 25 ml DMF geloest und mit 0.65 g (10 mmol) Natriumazid und 0.53 g (10 mmol) Ammoniumchlorid in einem Stahlautoklaven versetzt. Man laeßt 24 h bei 95°C und 150 bar Stickstoffdruck reagieren. Anschließend wird das DMF abdestilliert, der Rueckstand mit 2 M NaOH versetzt und mit Essigester extrahiert. Die waessrige Phase wird mit 2 M HCl sauer gestellt und mit Essigester extrahiert.

Nach dem Eindampfen wird der Rueckstand mit Cyclohexan ausgekocht und man erhaelt 0.22 g ( 28 % Ausbeute) der Titelverbindung vom Schmelzpunkt 96 - 7°C.

Beispiel 8

N-Hydroxy-S-[3-(2-pentyl-phenoxy)propyl]-3-mercaptopropionsaeureamid

2.08 g (15 mmol) Hydroxylamin-Hydrochlorid werden in 30 ml Methanol geloest und mit 45 ml 1 M Natriummethylatloesung versetzt. Nach 30 min werden 4.87 g (15 mmol) S-[3-(2-Pentyl-phenoxy)propyl]-3-mercaptopropionsaeuremethylester in 30 ml Methanol in 15 min zugetropft, und anschließend 4 h unter Rueckfluß erhitzt.

Man filtriert vom Salz ab und dampft die Loesung ein. Anschließend wird mit Essigester geloest, und wie in Beispiel 1 weiter verfahren.

Es wurden 2.0 g (41 % Ausbeute) der Titelverbindung als Oel erhalten.

Beispiel 9

In analoger Weise wie in Beispiel 8 wurden erhalten

| Bezeichnung | Ausbeute % | Schmp. (°C) Lsg.mittel |
|---|---|---|
| a) N-Hydroxy-S-[3-(3-<1-hydroxy-hexyl>-phenoxy)propyl]-4-mercaptobutter-saeureamid | 54 | Oel |
| b) N-Hydroxy-S-[3-(3-<1-hydroxy-hexyl>phenoxy)-2-hydroxy-propyl]-4-mercaptobuttersaeureamid | 36 | Oel |
| c) N-Hydroxy-S-[4-(3-<1-hydroxy-hexyl>-phenoxy)butyl]-3-mercaptopropion-saeureamid | 20 | Oel |
| d) N-Hydroxy-S-[4(3-<1-hydroxy-hexyl>-phenoxy)-but-2-enyl]-3-mercapto-propionsaeureamid | 41 | Oel |
| e) N-Hydroxy-S-[3-(3-pentylphenoxy)-propyl]-3-mercaptopropionsaeure-amid | 32 | Oel |

Beispiel 10

S-[3-(4-Acetyl-3-hydroxy-2-propyl-phenoxy)propyl]-2-mercaptoethanol

4.68 g (60 mmol) 2-Mercaptoethanol werden mit 60 ml 1 M Natriummethylatloesung 10 min bei Raumtemperatur geruehrt. Anschließend werden 9.19 g 3-(4-Acetyl-3-hydroxy-2-propyl-phenoxy)propylbromid zugegeben und 5 h unter Rueckfluß gekocht. Anschließend wird eingedampft und mit Wasser und Essigester versetzt. Der Essigester wird nacheinander mit 2 M NaOH und 2 M HCl ausgeschuettelt, getrocknet und eingedampft.

Nach Umkristallisieren aus Diethylether/Ligroin erhaelt man 6.4 g (68 % Ausbeute) der Titelverbindung vom Schmp. 72 - 4°C.

Beispiel 11

S-(3-(4-Acetyl-3-hydroxy-2-propyl-phenoxy)propyl]-2-mercaptoethylbromid

1.63 g (5 mmol) S-[3-(4-Acetyl-3-hydroxy-2-propyl-phenoxy)-propyl]-2-mercaptoethanol werden in 15 ml Methylenchlorid geloest und mit 2.07 g (6.25 mmol) Tetrabrommethan versetzt. Man kuehlt auf -10°C ab und versetzt protionsweise mit 1.96 g (7.5 mmol) Triphenylphosphin. Es wird noch 15 min nachgeruehrt, mit Ether versetzt und vom Niederschlag abgesaugt.

Der Ether wird abgedampft und der Rueckstand mit Hexan versetzt. Nach Abfiltrieren des Niederschlags und Abdampfen des Hexans werden 1.7 g (91 % Ausbeute) der Titelverbindung vom Schmelzpunkt 74 - 5°C erhalten.

Beispiel 12

S-[3-(4-Acetyl-3-hydroxy-2-propyl-phenoxy)propyl]-2-mercaptoethylrhodanid

1.0 g (2,8 mmol) S-[3-(4-Acetyl-3-hydroxy-2-propyl-phenoxy)-propyl]-2-mercaptoethylbromid und 5.0 g Kaliumthiocyanat werden in 25 ml Aceton 2 h unter Rueckfluß erhitzt.

Anschließend wird der Niederschlag abfiltriert, die Loesung eingedampft und der Rueckstand mit Diethylether verruehrt.

Nach Eindampfen des Ethers erhaelt man 0.7 g (71 % Ausbeute) der Titelverbindung als Oel.

Beispiel 13

S-[3-(4-Acetyl-3-hydroxy-2-allyl-phenoxy)propyl]-3-mercaptopropionitril

1.6 g (6.3 mmol) 3-(3-Mercaptopropionitril)-benzoisothiazol-S,S-dioxyd werden unter Stickstoff in 10 ml Acetonitril suspendiert. Man gibt 0.52 ml Piperidin zu und ruehrt 20 min bei Raumtemperatur. Anschließend werden 0.92 g DBU und 1.87 g (6 mmol) 3-(4-Acetyl-3-hydroxy-2-allyl-phenoxy)propylbromid zugegeben und 30 min bei Raumtemperatur geruehrt.

Nach dem Eindampfen wird der Rueckstand in Essigester geloest, mit 2 M NaOH ausgeschuettelt, neutral gewaschen und nach Trocknen eingedampft.

Nach Auskochen mit Ligroin und Eindampfen werden 1.2 g (63 % Ausbeute) der Titelverbindung als Oel erhalten.

Beispiel 14

In analoger Weise wie in Beispiel 13 wurden erhalten

| | Bezeichnung | Ausbeute % | Schmp. (°C) Lsg.mittel |
|---|---|---|---|
| a) | S-[3-(4-Acetyl-3-hydroxy-2-propyl-phenoxy)propyl]-6-mercaptohexannitril | 55 | Oel |
| b) | S-[3-(4-Acetyl-3-hydroxy-2-allyl-phenoxy)propyl]-mercaptoacetonitril | 48 | 71 (Ligroin) |
| c) | S-[3-(4-Acetyl-3-hydroxy-2-propyl-phenoxy)propyl]-3-mercaptopropionitril | 49 | 42 ($CH_2Cl_2$) |
| d) | S-[3-(2-Pentyl-phenoxy)propyl]-3-mercaptopropionitril | 60 | Oel |

Beispiel 15

Analog zu Beispiel 2 t, jedoch unter Einsatz von O-Trimethylsilyl-cyclohexylhydroxylamin, wurde erhalten:

| Bezeichnung | Ausbeute % | Schmp. (°C) Lsg.mittel |
|---|---|---|
| N-Hydroxy-N-cyclohexyl-S-[3-(4-acetyl-3-hydroxy-2-propyl-phenoxy)propyl]-3-mercaptopropionsaeureamid | 36 | 124-5 (Ligroin/ Aceton |

Die Zwischenverbindungen wurden wie folgt hergestellt:

a) [3-(1-Hydroxy-hexyl)phenoxy-methyl]-oxiran

19.4 g (0.1 mol) 3-(1-Hydroxyhexyl)phenol und 28.6 g Epichlorhydrin werden in 25 ml Ethanol geloest und zum Sieden erhitzt. Dann wird eine Loesung von 6.6 g KOH in 3 ml $H_2O$ und 25 ml Ethanol innerhalb von 15 Minuten zugetropft. Man laeßt 2 h unter Rueckfluß kochen, destilliert das Loesungsmittel ab und nimmt den Rueckstand in Wasser auf. Man extrahiert mit Essigester, wäscht den Essigester 2 mal mit Natronlauge und Wasser, trocknet, dampft ein und erhaelt 22.4 g (89 % d. Theorie) der Titelverbindung als Oel.

b) 1-Brom-3-[3-(1-Hydroxy-hexyl)phenoxy]-propan

19.4 g (0.1 mol) 3-(1-Hydroxy-hexyl)-phenol und 80.4 g (0.4 mol) Dibrompropan werden in 150 ml Methylethylketon geloest und auf 80°C erhitzt. Innerhalb 1 h werden 15.2 g $K_2CO_3$ zugegeben. Man laeßt 8 h bei 80°C ruehren, filtriert den Rueckstand ab und dampft das Loesungsmittel und das ueberschuessige Dibrompropan ab. Der Rueckstand wird in Essigester aufgenommen, 2 mal mit NaOH extrahiert, neutral gewaschen, getrocknet und eingedampft. Man erhaelt 25.8 g (82 % d. Theorie) der Titelverbindung als Oel.

c) In analoger Weise, wie unter b) beschrieben, wurden erhalten:

| Bezeichnung | Ausbeute % | Schmp. (°C) Lsg.mittel |
|---|---|---|
| 1-Brom-4-[3-(1-hydroxy-hexyl)phenoxy]-butan | 89 | Oel |
| 1-Chlor-4-[3-(1-hydroxyhexyl)phenoxy]-but-2-en | 85 | Oel |

d) S-[3-(3-<1-Hydroxy-hexyl>phenoxy)-2-hydroxy-propyl]-4-mercaptobuttersaeureethylester

9.42 g (0.03 mol) S-(Benzoisothiazol-3-yl-1,1-dioxyd)-4-mercaptobuttersaeureethylester werden in 60 ml Acetonitril geloest. Unter $N_2$ werden 2.5 ml Piperidin zugegeben und 20 Minuten bei Raumtemperatur geruehrt. Anschließend wird eine Loesung aus 5.52 g DBU und 7.5 g (0.03 mol) [3-(1-Hydroxy-hexyl)phenoxy-methyl]-oxiran in 50 ml Acetonitril zugetropft. Nach 1 h bei Raumtemperatur wird das Acetonitril abdestilliert, der Rueckstand in Essigester aufgenommen, mit 2 M NaOH ausgeschuettelt, mit $H_2O$ neutral gewaschen, getrocknet und eingedampft. Der Rueckstand wird mit Ether verruehrt, vom Unloeslichem abfiltriert und eingedampft. Man erhaelt 10.0 g (84 % d. Theorie) der Titelverbindung als Oel.

e) In analoger Weise,wie unter d) beschrieben,wurden erhalten:

| Bezeichnung | Ausbeute % | Schmp. (°C) Lsg.mittel |
|---|---|---|
| S-[3-(3-<1-Hydroxy-hexyl>phenoxy)-propyl]-4-mercaptobuttersaeureethylester | 90 | Oel |

f) S-[3-(3-<1-Hydroxy-hexyl>phenoxy)-2-hydroxy-propyl]-4-mercaptobuttersaeure

10.0 g S-[3-(3-<1-Hydroxy-hexyl>phenoxy)-2-hydroxy-propyl]-4-mercaptobuttersaeureethylester wird 2 h bei 50°C mit 50 ml Methanol und 50 ml 2 M NaOH geruehrt. Anschließend wird das Loesungsmittel abgezogen, in Wasser aufgenommen und 3 mal mit Essigester extrahiert. Die Wasserphase wird mit 2 M HCl angesaeuert und 3 mal mit Essigester extrahiert. Die org. Phase wird neutral gewaschen, getrocknet und eingedampft. Man erhaelt 7.4 g (80 % d. Theorie) der Titelverbindung als Oel.

g) In analoger Weise, wie unter f) beschrieben, wurde erhalten:

| Bezeichnung | Ausbeute % | Schmp. (°C) Lsg.mittel |
|---|---|---|
| S-[3-(3-<1-Hydroxy-hexyl>phenoxy)-propyl]-4-mercaptobuttersaeure | 85 | Oel |

h) S-[4-(3-<1-Hydroxy-hexyl>phenoxy)-but-2-enyl]-3-mercaptopropionsaeure

9.15 ml (0.105 mol) 3-Mercaptopropionsaeure werden in 70 ml Methanol geloest und mit 315 ml 1 M Natriummethylat-Loesung 10 Minuten bei Raumtemperatur unter Stickstoff geruehrt. Anschließend werden 19.79 g (0.07 mol) 1-Chlor-4-[3-(1-hydroxyhexyl)phenoxy]-but-2-en in 70 ml Methanol in 15 min zugetropft. Man laeßt 3 h bei Raumtemperatur nachruehren und zieht dann das Loesungsmittel ab. Der Rueckstand wird in Diethylether aufgenommen und mehrmals mit 2 M NaOH extrahiert. Die waessrige Loesung wird mit 2 M HCl angesaeuert und die gesuchte Verbindung mit Methylenchlorid extrahiert. Nach Trocknen und beziehen des Loesungsmittels erhaelt man 22.5 g (91 % d. Theorie) der Titelverbindung als Oel.

i) In analoger Weise, wie unter h) beschrieben, erhaelt man:

| Bezeichnung | Ausbeute % | Schmp. (°C) Lsg.mittel |
|---|---|---|
| S-[4-(3-<1-Hydroxy-hexyl>phenoxy)-butyl]-3-mercaptopropionsaeure | 90 | Oel |

## Patentansprüche

1. Verbindungen der allgemeinen Formel I

$$Ar{-}O{-}A{-}\overset{(O)_n}{\overset{|}{S}}{-}B{-}R \qquad (I),$$

in der

Ar einen Phenylrest, der gegebenenfalls ein-, zwei- oder dreifach durch $C_1$-$C_6$-Alkyl, $C_2$-$C_6$-Alkenyl, $C_1$-$C_6$-Alkoxy, $C_1$-$C_6$-Alkylthio, $C_1$-$C_6$-Alkanoyl, Hydroxyl, Hydroxy-$C_1$-$C_6$-alkyl, Carboxyl, $C_1$-$C_6$-Alkoxycarbonyl, Carbamoyl, Nitro, Amino und Halogen substituiert sein kann,

A einen geradkettigen oder verzweigten, gesättigten oder ungesättigten $C_1$-$C_8$-Alkylenrest, der gegebe-

nenfalls durch eine Hydroxylgruppe substituiert sein kann,

n die Zahl 0, 1 oder 2,

B einen geradkettigen oder verzweigten, gesättigten oder ungesättigten $C_1$-$C_8$-Alkylenrest und

R 5-(1H)-Tetrazolyl, -CO-NH-Tetrazolyl, -CO-N(OH)-$R_1$, 5-(1H)-Tetrazolylthio, -C(=NH)-N(OH)-$R_1$ oder -CO-O-NH-$R_1$ bedeuten, wobei

$R_1$ Wasserstoff, $C_1$-$C_6$-Alkyl, das gegebenenfalls durch $C_3$-$C_7$-Cycloalkyl substituiert sein kann, $C_2$-$C_6$-Alkenyl, $C_3$-$C_7$-Cycloalkyl, Phenyl-$C_1$-$C_6$-alkyl und Phenyl darstellen, wobei der aromatische Teil durch $C_1$-$C_6$-Alkyl, $C_2$-$C_6$-Alkenyl, $C_1$-$C_6$-Alkoxy, $C_1$-$C_6$-Alkylthio, $C_1$-$C_6$-Alkanoyl, Hydroxyl, Hydroxy-$C_1$-$C_6$-alkyl, Carboxyl, $C_1$-$C_6$-Alkoxycarbonyl, Carbamoyl, Nitro, Amino und Halogen substituiert sein kann,

sowie deren pharmakologisch verträglichen Salze.

2. Verbindungen der allgemeinen Formel I gemäß Anspruch 1, in der

Ar einen Phenylrest, der gegebenenfalls durch Propyl, Pentyl, Allyl, Methoxy, Ethoxy, Acetyl, Hydroxyl, 1-Hydroxy-hexyl und Halogen ein- bis dreifach substituiert sein kann,

A einen Methylen-, Ethylen-, Propylen-, Butylen-, Pentylen-, But-2-enylen-, But-2-inylen- und 2-Hydroxypropylenrest,

n die Zahl 0,

B einen Methylen-, Ethylen-, Propylen-, Butylen- oder Pentylenrest und

R eine 5-(1H)-Tetrazolyl-, -CO-NH-Tetrazolyl-, -CO-N(OH)-$R_1$, 5-(1H)-Tetrazolylthio- und -CO-O-NH-$R_1$-Gruppe bedeuten,

wobei $R_1$ Wasserstoff, eine Methyl-, Ethyl-, Propyl-, Cyclohexyl- und Phenylgruppe darstellt,

sowie deren pharmakologisch verträglichen Salze.

3. Verbindungen der allgemeinen Formel I gemäß Anspruch 1, in der

Ar einen Phenylrest, der durch Propyl, Allyl, Hydroxy, Acetyl und 1-Hydroxy-hexyl substituiert ist,

A einen Propylen-, Butylen-, But-2-enylen- und 2-Hydroxypropylen-Rest,

n die Zahl 0

B einen Methylen-, Ethylen-, Propylen- und Pentylenrest und

R eine N-Hydroxy-N-Methyl-aminocarbonyl-, 5-Tetrazolylaminocarbonyl-, 5-Tetrazolyl-, 5-Tetrazolylthio- oder N-Hydroxy-aminocarbonylgruppe

bedeuten, sowie deren pharmakologisch verträglichen Salze.

4. Verfahren zur Herstellung von Verbindungen der allgemeinen Formel I

$$Ar-O-A-\overset{(O)_n}{\overset{|}{S}}-B-R \qquad (I),$$

gemäß Anspruch 1, 2 oder 3

sowie deren pharmakologischen Salze, dadurch gekennzeichnet, daß man

a) eine Verbindung der allgemeinen Formel II

Ar-O-A-X (II),

mit einer Verbindung der allgemeinen Formel III

Y-B-G (III),

worin

Ar, A und B die oben genannte Bedeutung haben, einer der beiden Reste X oder Y die Gruppe -SH, der andere einen mit der -SH-Gruppe reagierenden Rest darstellt und G für den oben genannten Rest R oder eine Hydroxylgruppe steht oder

b) eine Verbindung der allgemeinen Formel IV

Ar-O-A-S-B-COOH (IV),

in der

Ar, A und B die oben genannte Bedeutung haben, oder ein Carbonsäurehalogenid, Carbonsäureester oder gemischtes Carbonsäureanhydrid hiervon mit 5-Amino-(1H)-tetrazol oder mit einer Verbindung der allgemeinen Formel V

HN(OH)-$R_1$ (V),

oder einem O-silylierten Derivat davon, in welcher $R_1$ die oben genannte Bedeutung hat, umsetzt und anschließend gewünschtenfalls

eine für G stehende Hydroxylgruppe, beispielsweise nach Überführung in ein Halogenid, Mesylat oder Tosylat in einen Rest R überführt,

eine Verbindung der Formel I, in der R für eine Cyano- oder Rhodanogruppe steht, mit Stickstoffwasserstoffsäure oder einem Alkaliazid und einer protonenliefernden Substanz umsetzt, und so Verbindungen erhält, in

denen R die übrigen Bedeutungen besitzt,
das Schwefelatom oxidiert und das so erhaltene Reaktionsprodukt in ein pharmakologisch verträgliches Salz überführt.

5. Arzneimittel enthaltend eine oder mehrere Verbindungen gemäß Anspruch 1, 2 oder 3 sowie gegebenenfalls pharmakologische Träger- und/oder Hilfsstoffe.

6. Verbindungen gemäß Anspruch 1, 2 oder 3 zur Bekämpfung von allergischen Krankheiten.

7. Verwendung von Verbindungen gemäß Anspruch 1, 2 oder 3 zur Herstellung von Arzneimitteln.

8. Verfahren zur Herstellung von Arzneimitteln, die eine oder mehrere Verbindungen der Formel I gemäß Anspruch 1, 2 oder 3 enthalten, dadurch gekennzeichnet, daß man eine Verbindung der Formel I mit pharmakologischen Träger- und/oder Hilfsstoffen mischt und zu applizierbaren Arzneimitteln verarbeitet.

## Claims

1. Compounds of the general formula I

$$\text{Ar-O-A-}\overset{(O)_n}{\overset{|}{\text{S}}}\text{-B-R} \qquad (I)$$

in which

Ar signifies a phenyl radical which can possibly be substituted one, two or three times by $C_1$-$C_6$-alkyl, $C_2$-$C_6$-alkenyl, $C_1$-$C_6$-alkoxy, $C_1$-$C_6$-alkylthio, $C_1$-$C_6$-alkanoyl, hydroxyl, hydroxy-$C_1$-$C_6$-alkyl, carboxyl, $C_1$-$C_6$-alkoxycarbonyl, carbamoyl, nitro, amino and halogen,

A a straight-chained or branched, saturated or unsaturated $C_1$-$C_8$-alkylene radical which can possibly be substituted by a hydroxyl group,

n the number 0, 1 or 2,

B a straight-chained or branched, saturated or unsaturated $C_1$-$C_8$-alkylene radical and

R 5-(1H)-tetrazolyl, -CO-NH-tetrazolyl, -CO-N(OH)-$R_1$, 5-(1H)-tetrazolylthio, -C(=NH)-N(OH)-$R_1$ or -CO-O-NH-$R_1$,

whereby $R_1$ represents hydrogen, $C_1$-$C_6$-alkyl, which can possibly be substituted by $C_3$-$C_7$-cycloalkyl, $C_2$-$C_6$-alkenyl, $C_3$-$C_7$-cycloalkyl, phenyl-$C_1$-$C_6$-alkyl and phenyl, whereby the aromatic moiety can be substituted by $C_1$-$C_6$-alkyl, $C_2$-$C_6$-alkenyl, $C_1$-$C_6$-alkoxy, $C_1$-$C_6$-alkylthio, $C_1$-$C_6$-alkanoyl, hydroxyl, hydroxy-$C_1$-$C_6$-alkyl, carboxyl, $C_1$-$C_6$-alkoxycarbonyl, carbamoyl, nitro, amino and halogen,

as well as their pharmacologically acceptable salts.

2. Compounds of the general formula I according to claim 1, in which

Ar signifies a phenyl radical which can possibly be substituted one to three times by propyl, pentyl, allyl, methoxy, ethoxy, acetyl, hydroxyl, 1-hydroxyhexyl and halogen,

A a methylene, ethylene, propylene, butylene, pentylene, but-2-enylene, but-2-ynylene and 2-hydroxypropylene radical,

n the number 0,

B a methylene, ethylene, propylene, butylene or pentylene radical and

R a 5-(1H)-tetrazolyl, -CO-NH-tetrazolyl, -CO-N(OH)-$R_1$, 5-(1H)-tetrazolylthio and -CO-O-NH-$R_1$ group, whereby

$R_1$ represents hydrogen, a methyl, ethyl, propyl, cyclohexyl and phenyl group,

as well as their pharmacologically acceptable salts.

3. Compounds of the general formula I according to claim 1, in which

Ar signifies a phenyl radical which is substituted by propyl, allyl, hydroxyl, acetyl and 1-hydroxyhexyl,

A a propylene, butylene, but-2-enylene and 2-hydroxypropylene radical,

n the number 0,

B a methylene, ethylene, propylene and pentylene radical and

R an N-hydroxy-N-methylaminocarbonyl, 5-tetrazolylaminocarbonyl, 5-tetrazolyl, 5-tetrazolylthio or N-hydroxyaminocarbonyl group,

as well as their pharmacologically acceptable salts.

4. Process for the preparation of compounds of the general formula I

$$\overset{(O)_n}{\overset{|}{\text{Ar-O-A-S-B-R}}} \qquad (I)$$

according to claim 1, 2 or 3, as well as of their pharmacological salts, characterised in that one reacts

a) a compound of the general formula II

Ar-O-A-X (II)

with a compound of the general formula III

Y-B-G (III)

wherein

Ar, Y and B have the above-given meaning, one of the two residues X or Y represents the group -SH, the other a residue reacting with the -SH group and G stands for the above-mentioned residue R or a hydroxyl group, or

b) a compound of the general formula IV

Ar-O-A-S-B-COOH (IV)

in which

Ar, A and B have the above-given meaning, or a carboxylic acid halide, carboxylic acid ester or mixed carboxylic acid anhydride thereof, with 5-amino-(1H)-tetrazole or with a compound of the general formula V

$HN(OH)-R_1$ (V)

or with an 0-silylated derivative thereof, in which $R_1$ has the above-given meaning,

and subsequently, if desired, converts a hydroxyl group standing for G, for example after conversion into a halide, mesylate or tosylate, into a radical R,

reacts a compound of the formula I, in which R stands for a cyano or rhodano group, with hydrazoic acid or an alkali metal azide and a proton-providing substance and thus obtains compounds in which R possesses the other meanings,

oxidises the sulphur atom and converts the so-obtained reaction product into a pharmacologically acceptable salt.

5. Medicaments containing one or more compounds according to claim 1, 2 or 3, as well as possibly pharmacological carrier and/or adjuvant materials.

6. Compounds according to claim 1, 2 or 3 for the combating of allergic diseases.

7. Use of compounds according to claim 1, 2 or 3 for the production of medicaments.

8. Process for the production of medicaments which contain one or more compounds of the formula I according to claim 1, 2 or 3, characterised in that one mixes a compound of the formula I with pharmacological carrier and/or adjuvant materials and works up to administerable medicaments.

**Revendications**

1. Composés de formule générale I

$$\overset{(O)_n}{\overset{|}{\text{Ar-O-A-S-B-R}}} \qquad (I),$$

dans laquelle

Ar représente un groupe phényle, qui peut être éventuellement substitué une, deux ou trois fois par un groupe alkyle en $C_1$-$C_6$, un groupe alcényle en $C_2$-$C_6$, un groupe alcoxy en $C_1$-$C_6$, un groupe alkylthio en $C_1$-$C_6$, un groupe alcanoyle en $C_1$-$C_6$, un groupe hydroxyle, un groupe hydroxyalkyle en $C_1$-$C_6$, un groupe carboxyle, alcoxycarbonyle en $C_1$-$C_6$, carbamoyle, nitro, amino et halogéno,

A représente un groupe alkylène en $C_1$-$C_8$, saturé ou insaturé, à chaîne droite ou ramifiée, qui peut être éventuellement substitué par un groupe hydroxyle,

n vaut 0, 1 ou 2,

B représente un groupe alkylène en $C_1$-$C_8$, saturé ou insaturé, à chaîne droite ou ramifiée, et

R représente un groupe 5-(1H)-tétrazolyle, -CO-NH-tétrazolyle, $-CO-N(OH)-R_1$, 5-(1H)-tétrazolylthio, -$C(=NH)-N(OH)-R_1$ ou -CO-O-NH-R 1,

$R_1$ représentant l'hydrogène, un groupe alkyle en $C_1$-$C_6$, qui peut être éventuellement substitué par un groupe cycloalkyle en $C_3$-$C_7$, un groupe alcényle en $C_2$-$C_6$, un groupe cycloalkyle en $C_3$-$C_7$, un groupe phénylalkyle en $C_1$-$C_6$ et un groupe phényle, la partie aromatique pouvant être substituée par un groupe alkyle en $C_1$-$C_6$, alcényle en $C_2$-$C_6$, alcoxy en $C_1$-$C_6$, alkylthio en $C_1$- $C_6$, alcanoyle en $C_1$-$C_6$, hydroxyle, hydroxyalkyle en $C_1$- $C_6$, carboxy, alcoxycarbonyle en $C_1$-$C_6$, carbamoyle, nitro, amino et halogéno,
ainsi que leurs sels pharmacologiquement acceptables.

2. Composés de formule générale I selon la revendication 1,
dans laquelle

Ar représente un groupe phényle, qui peut être éventuellement substitué une à trois fois par un groupe propyle, pentyle, allyle, méthoxy, éthoxy, acétyle, hydroxyle, 1-hydroxyhexyle et halogène,

A représente un groupe méthylène, éthylène, propylène, butylène, pentylène, but-2-énylène, but-2-inylène et 2-hydroxypropylène,

n vaut 0,

B représente un groupe méthylène, éthylène, propylène, butylène ou pentylène et

R représente un groupe 5-(1H)-tétrazolyle, -CO-NH-tétrazolyle, -CO-N(OH)-$R_1$, 5-(1H)-tétrazolylthio et -CO-O-NH-$R_1$,

$R_1$ représentant un groupe méthyle, éthyle, propyle, cyclohexyle et phényle,
ainsi que leurs sels pharmacologiquement acceptables.

3. Composés de formule générale I selon la revendication 1,
dans laquelle

Ar représente un groupe phényle, qui est substitué par un groupe propyle, allyle, hydroxyle, acétyle et 1-hydroxyhexyle,

A représente un groupe propylène, butylène, but-2-énylène et 2-hydroxypropylène,

n vaut 0,

B représente un groupe méthylène, éthylène, propylène et pentylène et

R représente un groupe N-hydroxy-N-méthylaminocarbonyle, 5-tétrazolylaminocarbonyle, 5-tétrazolyle-, 5-tétrazolylthio ou N-hydroxyaminocarbonyle,
ainsi que leurs sels pharmacologiquement acceptables.

4. Procédé de préparation de composés de formule générale I

$$Ar-O-A-\overset{(O)_n}{\overset{|}{S}}-B-R \qquad (I),$$

selon la revendication 1, 2 ou 3,
ainsi que leurs sels pharmacologiquement acceptables, caractérisé en ce que
a) on fait réagir un composé de formule générale II

Ar-O-A-X (II),

avec un composé de formule générale III

Y-B-G (III),

dans laquelle

Ar, A et B ont les significations mentionnées ci-dessus, l'une des deux groupes X ou Y représente le groupe -SH, l'autre un groupe réagissant avec le groupe -SH, et G représente le groupe R mentionné ci-dessus ou un groupe hydroxyle, ou
b) on fait réagir un composé de formule générale IV

Ar-O-A-S-B-COOH (IV),

dans laquelle

Ar, A et B ont les significations mentionnées ci-dessus, ou un halogénure d'acide carboxylique, un ester d'acide carboxylique ou un anhydride mixte d'acide carboxylique, avec le 5-amino-(1H)-tétrazole ou avec un composé de formule générale V

HN(OH)-$R_1$ (V),

dans laquelle $R_1$ a la signification mentionnée ci-dessus, ou avec un dérivé O-silylé de ce dernier composé,
et ensuite, le cas échéant,
on transforme un groupe hydroxyle représenté par G, par exemple après transformation en un halogénure, un mésylate ou un tosylate, en un groupe R,
on fait réagir un composé de formule I, dans laquelle R représente un groupe cyano ou rhodano, avec l'acide

hydroazoïque ou avec un azoture alcalin et une substance fournissant des protons, et l'on obtient ainsi des composés dans lesquels R a les significations restantes,
on oxyde l'atome de soufre et l'on transforme le produit de réaction ainsi obtenu en un sel pharmacologiquement acceptable.

5. Médicament contenant un ou plusieurs composés selon la revendication 1, 2 ou 3, ainsi qu'éventuellement des véhicules et/ou adjuvants pharmacologiquement acceptables.

6. Composés selon la revendication 1, 2 ou 3, pour lutter contre des affections allergiques.

7. Utilisation des composés selon la revendication 1, 2 ou 3, pour la fabrication de médicaments.

8. Procédé pour la préparation de médicaments contenant un ou plusieurs composés de formule I selon la revendication 1, 2 ou 3, caractérisé en ce que l'on mélange un composé de formule I avec des véhicules et/ou adjuvants pharmacologiques et on les transforme en médicaments que l'on peut administrer.